# EUROPEAN PATENT APPLICATION

(11) **EP 4 342 486 A1**
(43) Date of publication of application: **27.03.2024**
(21) Application number: 22196673.2
(22) Date of filing: 20.09.2022
(51) Int. Cl.: A61K 38/22, A61K 38/28, A61P 3/10, A61K 47/34

(54) **COMPOSITIONS COMPRISING AT LEAST AN INSULIN AND AN AMYLIN RECEPTOR AGONIST FOR TREATING DIABETES, IN A PATIENT/PERSON HAVING A BMI OF MORE THAN 28 KG/M² AND/OR AN HBA1C OF MORE THAN 7.6 %**

(71) Applicant: Adocia, 69003 Lyon (FR)
(72) Inventor: SOULA, Olivier, 6930 Meyzieu (FR)
(74) Representative: Tripoz, Inès

(57) **Abstract**

The invention relates to a composition comprising an insulin and an amylin receptor agonist for treating diabetes, in particular type 1 diabetes, in a patient/person having a BMI of more than 28, in particular of more than or equal to 30 and/or an HbAlc of more than 7.6 %.

In an embodiment the insulin is human insulin A21G or insulin or lispro A21G or insulin aspart A21G or human insulin or insulin lispro or insulin aspart.

In an embodiment the composition comprises from 40 to 500 U/ml of insulin.

In an embodiment the amylin receptor agonist is a short-acting amylin receptor agonist, in particular pramlintide.

## Description

The invention relates to the treatment of diabetes, in particular Type 1 diabetes, by a composition comprising at least a prandial insulin and an amylin receptor agonist, in a patient having a BMI of more than 28 kg/m² and/or an HbA1c of more than 7.6 %.

Overweight and obesity are getting more and more common in diabetic patients, and also in T1D patients. However, there is currently no approved T1D treatment involving insulin (which is in many case the treatment of choice) which allows weight loss. Such a treatment would greatly improve the quality of life of these persons.

Surprisingly, the applicant has demonstrated that a composition containing an insulin in combination with an amylin receptor agonist for the treatment of diabetes leads to excellent results in terms of loss of weight on patients having a BMI (Body Mass Index) > 28 kg/m² and/or an HbA1c > 7.6 %.

In an embodiment the patients have a BMI of more than 28 kg/m².

In an embodiment the patients have a BMI of more than or equal to 30 kg/m².

In an embodiment the patients have an HbA1c of more than 7.6 %

In an embodiment the insulin is a prandial insulin.

In an embodiment, the insulin is a prandial insulin A21G. Prandial insulin "A21G" means an insulin having the same sequence than human insulin but in which the position 21 on the A chain is a glycine residue.

The prandial insulin A21G can be chosen in the group consisting of:
- human insulin A21G, which has the sequence of human insulin in which the Asparagine in position A21 is replaced by Glycine,
- insulin lispro A21G, which has the sequence of insulin lispro in which the Asparagine in position A21 is replaced by Glycine, also defined as human insulin A21G, B28K, B29P, and
- insulin aspart A21G, which has the sequence of insulin aspart in which the Asparagine in position A21 is replaced by Glycine, also defined as human insulin A21G, B28D.

In an embodiment, the prandial insulin is a human insulin A21G.

In an embodiment, the prandial insulin is insulin lispro A21G.

In an embodiment, the prandial insulin is a insulin aspart A21G.

In an embodiment, the prandial insulin is chosen in the group consisting of human insulin, insulin lispro, insulin aspart and insulin glulisin.

In an embodiment, the insulin is human insulin.

In an embodiment, the insulin is insulin lispro.

In an embodiment, the insulin is insulin aspart.

In an embodiment, the insulin is insulin glulisin.

In an embodiment, the composition comprises from 40 U/ml to 500 U/ml of insulin, in particular of prandial insulin.

In an embodiment, the composition comprises from 100 U/ml to 400 U/ml of insulin, in particular of prandial insulin.

In an embodiment, the composition comprises from 100 U/ml to 200 U/ml of insulin, in particular of prandial insulin.

In an embodiment, the composition comprises 100 U/ml of insulin, in particular of prandial insulin.

In an embodiment, the composition comprises 200 U/ml of insulin, in particular of prandial insulin.

In this specification 100 U of human insulin A21G, also called M1, corresponds to 3.45 mg.

In this specification 100 U of insulin lispro A21G, also called L1, corresponds to 3.45 mg.

In this specification 100 U of insulin aspart A21G, also called A1, corresponds to 3.45 mg.

In an embodiment, the amylin receptor agonist is a short acting amylin receptor agonist.

In an embodiment they have a half-life of less than 8 h.

This half-life is the apparent half-life of elimination after subcutaneous injection in humans.

In an embodiment they have a half-life of less than 5 hours.

In an embodiment they have a half-life of less than 4 hours.

In an embodiment they have a half-life of less than 3 hours.

In an embodiment, the short acting amylin receptor agonist is pramlintide.

In an embodiment, the composition comprises from 0.2 mg/ml to 5 mg/ml of the amylin receptor agonist, in particular of pramlintide.

In an embodiment, the composition comprises from 0.4 mg/ml to 4 mg/ml of the amylin receptor agonist, in particular of pramlintide.

In an embodiment, the composition comprises from 0.5 mg/ml to 3 mg/ml of the amylin receptor agonist, in particular of pramlintide.

In an embodiment, the composition comprises from 0.5 mg/ml to 2 mg/ml of the amylin receptor agonist, in particular of pramlintide.

In an embodiment, the composition comprises 0.6 mg/ml of the amylin receptor agonist, in particular of pramlintide.

In an embodiment, the composition comprises 0.9 mg/ml of the amylin receptor agonist, in particular of pramlintide.

In an embodiment, the composition comprises 1.2 mg/ml of the amylin receptor agonist, in particular of pramlintide.

In an embodiment, the composition comprises from 0.3 to 2.5 mg of the amylin receptor agonist, in particular of pramlintide for 100 U of insulin.

In an embodiment, the composition comprises from 0.4 mg to 2 mg of the amylin receptor agonist, in particular of pramlintide, for 100 U of insulin.

In an embodiment, the composition comprises from 0.5 mg to 1.5 mg of the amylin receptor agonist, in particular of pramlintide, for 100 U of insulin.

In an embodiment, the composition comprises from 0.5 mg to 1 mg of the amylin receptor agonist, in particular of pramlintide, for 100 U of insulin.

In an embodiment, the composition comprises 0.6 mg of the amylin receptor agonist, in particular of pramlintide, for 100 U of insulin.

In an embodiment, the composition comprises 0.9 mg of the amylin receptor agonist, in particular of pramlintide, for 100 U of insulin.

In an embodiment, the composition comprises 1.2 mg of the amylin receptor agonist, in particular of pramlintide, for 100 U of insulin.

In an embodiment the composition is an aqueous solution.

In a further embodiment it is a clear aqueous solution.

In an embodiment the composition is in the form of injectable aqueous solution.

The compositions in the form of an injectable aqueous solution are clear solutions. By "clear solution", is meant compositions which meet the criteria described in the American and European pharmacopoeias regarding injectable solutions. In the US pharmacopoeia, the solutions are defined in section <1151> referring to injection (<1>) (referring to <788> according to USP 35 and specified in <788> according to USP 35 and in <787>, <788> and <790> USP 38 (beginning with August 1, 2014), according to USP 38). In the European pharmacopoeia, injectable solutions must meet the criteria given in sections 2.9.19 and 2.9.20.

In an embodiment the pH of the composition comprising insulin A21G is ranging from 3.0 to 4.4.

In an embodiment the pH of the composition comprising human insulin A21G is ranging from 3.5 to 4.2.

In an embodiment the pH of the composition comprising human insulin A21G is ranging from 3.8 to 4.2.

In an embodiment the pH of the composition comprising human insulin A21G is 4.0.

In an embodiment the pH of the composition comprising insulin lispro A21G is ranging from 3.6 to 4.4.

In an embodiment the pH of the composition comprising insulin lispro A21G is ranging from 3.6 to 4.0.

In an embodiment the pH of the composition comprising insulin lispro A21G is 3.8.

In an embodiment the pH of the composition comprising insulin aspart A21G is ranging from 3.0 to 4.0.

In an embodiment the pH of the composition comprising insulin aspart A21G is ranging from 3.2 to 3.6.

In an embodiment the pH of the composition comprising insulin aspart A21G is 3.5.

In an embodiment the pH of the composition is comprised from 6.0 to 8.0.

In an embodiment the pH of the composition is comprised from 6.4 to 7.8.

In one embodiment, the pH of the composition is comprised from 6.6 to 7.6.

In one embodiment, the pH of the composition is comprised from 6.8 to 7.4.

In an embodiment, when the pH of the composition is comprised between 6.0 and 8.0, in particular comprising insulin chosen from the group consisting of human insulin, insulin lispro, insulin aspart or insulin glulisin, the composition comprises a co-polyamino acid as defined in WO2019110788 and WO2019110797. The parts of this applications disclosing said co-polyamino acid being incorporated by reference.

In an embodiment, said copolyamino acid is bearing carboxylate charges and hydrophobic radicals Hy, said co-polyamino acid being constituted of glutamic or aspartic units and said hydrophobic radicals Hy chosen among the radicals according to formula X as defined below: 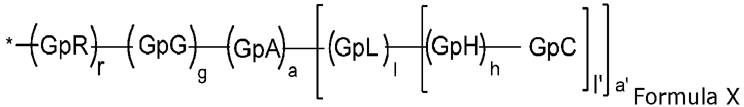 in which GpR is chosen among the radicals according to formulas VII, VII' or VII": 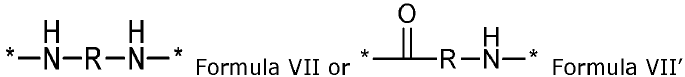 or 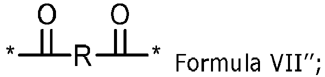
- Identical or different GpG and GpH are chosen among the radicals according to formulas XI or XI'; 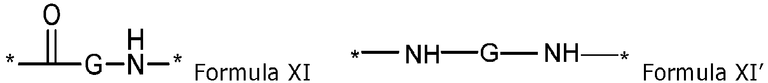
- GpA is chosen among the radicals according to formula VIII

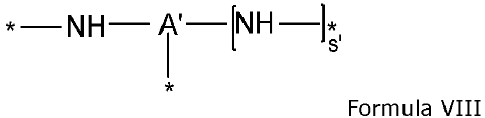

In which A' is chosen among the radicals according to formulas VIII', VIII" or VIII‴ 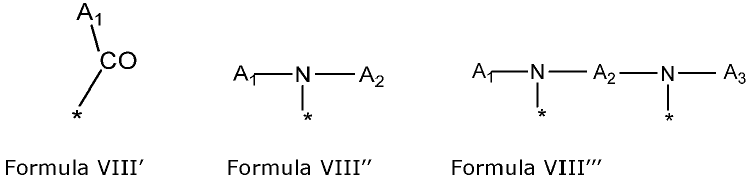
- -GpL is chosen among the radicals according to formula XII 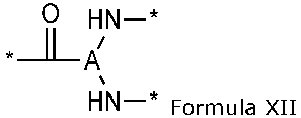
- GpC is a radical according to formula IX: 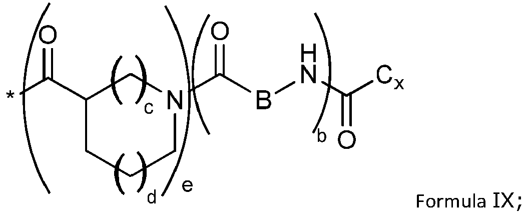
- ^{∗} indicate the attachment sites of the different groups bound by amide functions;
- a is an integer equal to 0 or to 1 and a' = 1 if a = 0 and a' = 1, 2 or 3 if a =1;
- a' is an integer equal to 1, to 2 or to 3;
- b is an integer equal to 0 or to 1;
- c is an integer equal to 0 or to 1, and if c is equal to 0, then d is equal to 1 or to 2;
- d is an integer equal to 0, to 1 or to 2;
- e is an integer equal to 0 or to 1;
- g is an integer equal to 0, to 1, to 2, to 3, to 4, to 5 or to 6;
- h is an integer equal to 0, to 1, to 2, to 3, to 4, to 5 or to 6, and at least one of g, h or I is different from 0;
- I is an integer equal to 0 or to 1 and I' = 1 if I = 0 and I' = 2 if I =1;
- r is an integer equal to 0, 1 or to 2, and
- s' is an integer equal to 0 or to 1;
- And if e is different from 0, then at least one of g, h or I is different from 0;
- And if a = 0, then I = 0;
- A, A₁, A₂ and A₃ identical or different, are linear or branched alkyl radicals comprising from 1 to 8 carbon atoms and, optionally, substituted by a radical from a saturated, unsaturated or aromatic ring;
- B is a radical ether or polyether, unsubstituted, comprising from 4 to 14 carbon atoms and 1 to 5 oxygen atoms, or a linear or branched alkyl radical, optionally comprising an aromatic ring, comprising from 1 to 9 carbon atoms.
- Cₓ is a monovalent, linear or branched, alkyl radical optionally comprising a cyclic part, in which x indicates the number of carbon atoms, and:
   ▪ When the hydrophobic radical -Hy bears 1 -GpC, then 9 ≤ x ≤ 25,
   ▪ When the hydrophobic radical -Hy bears 2 -GpC, then 9 ≤ x ≤ 15,
   ▪ When the hydrophobic radical -Hy bears 3 -GpC, then 7 ≤ x ≤ 13,
   ▪ When the hydrophobic radical -Hy bears 4 -GpC, then 7 ≤ x ≤ 11,
   ▪ When the hydrophobic radical -Hy bears at least 5 -GpC, then 6 ≤ x ≤ 11,
- G is a linear or branched divalent alkyl radical of 1 to 8 carbon atoms, said alkyl radical bearing one or more free carboxylic acid functions,
- R is a radical chosen from the group consisting of a divalent, linear or branched alkyl radical comprising from 1 to 12 carbon atoms, a divalent, linear or branched alkyl radical comprising from 1 to 12 carbon atoms bearing one or more -CONH₂ functions or an unsubstituted ether or polyether radical comprising from 4 to 14 carbon atoms and 1 to 5 oxygen atoms,
- The hydrophobic radical(s) -Hy according to formula X being bound to the PLG:
   ∘ via a covalent bond between a carbonyl of the hydrophobic radical -Hy and a nitrogen atom borne by the PLG, thus forming an amide function resulting from the reaction of an amine function borne by the PLG and an acid function borne by the precursor -Hy' of the hydrophobic radical -Hy, and
   ∘ via a covalent bond between a nitrogen atom of the hydrophobic radical -Hy and a carbonyl borne by the PLG, thus forming an amide function resulting from the reaction of an amine function of the precursor -Hy' of the hydrophobic radical -Hy and an acid function borne by the PLG,
- The ratio M between the number of hydrophobic radicals and the number of glutamic or aspartic units being comprised from 0 < M ≤ 0.5;
- When several hydrophobic radicals are borne by a co-polyamino acid, then they are identical or different,
- The degree of polymerization DP in glutamic or aspartic units for the PLG chains is comprised from 5 to 250;
- Free carboxylic acids being in the form of an alkaline cation salt chosen from the group consisting of Na⁺ and K⁺.

In an embodiment the precursor of the Hydrophobic graft is represented by Formula A

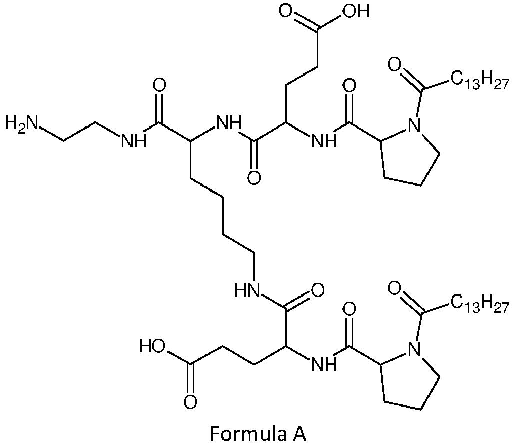

In an embodiment, the co-polyamino acid bearing carboxylate charges and hydrophobic radicals is chosen among the co-polyamino acids according to formula XXX below: 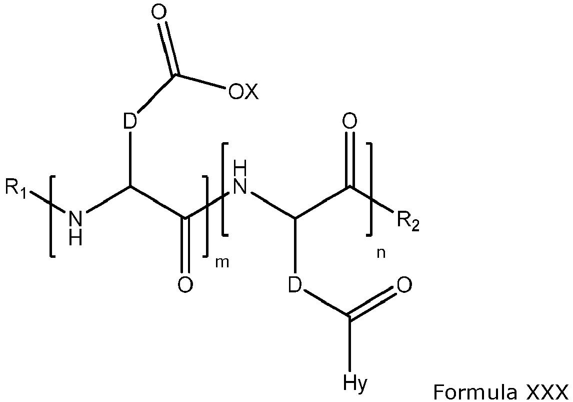 in which,
- D represents, independently, either a -CH₂- group (aspartic unit) or a - CH₂-CH₂- group (glutamic unit),
- Hy is a hydrophobic radical chosen among the hydrophobic radicals according to formula X,
- R₁ is a hydrophobic radical chosen among the hydrophobic radicals according to formula X in which r=0 or r=1 and GpR is a radical according to formula VII' or VII", or a radical chosen from the group consisting of a H, a linear acyl group in C2 to C10, a branched acyl group in C3 to C10, a benzyl, an end "amino acid" unit and a pyroglutamate,
- R₂ is a hydrophobic radical chosen among the hydrophobic radicals according to formula X or an -NR'R" radical, R' and R", identical or different, being chosen from the group consisting of H, the linear, branched or cyclic alkyls in C2 to C10, benzyl and said R' and R" alkyls may together form one or more saturated, unsaturated and/or aromatic rings and/or may comprise heteroatoms, chosen from the group consisting of O, N and S,
- X represents a H or a cationic entity chosen from the group comprising the metallic cations;
- n + m represents The degree of polymerization DP of the co-polyamino acid, that is the average number of monomeric units per co-polyamino acid chain and 5 ≤ n + m ≤ 250.

In an embodiment, the co-polyamino acid bearing carboxylate charges and hydrophobic radicals is chosen among the co-polyamino acids according to formula XXXb below: 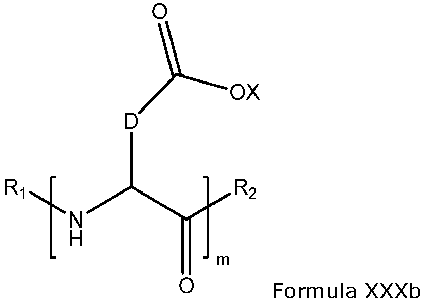 in which m, X, D, R₁ and R₂ as defined in claim 2 and at least R₁ or R₂ is a hydrophobic radical according to formula X.

In an embodiment the co-polyamino acid correspond to Formula XXXb where only R₁ is a hydrophobic radical according to formula X.

In an embodiment the co-polyamino acid correspond to Formula XXXb where only R₂ is a hydrophobic radical according to formula X.

In an embodiment the co-polyamino acid correspond to Formula B1' 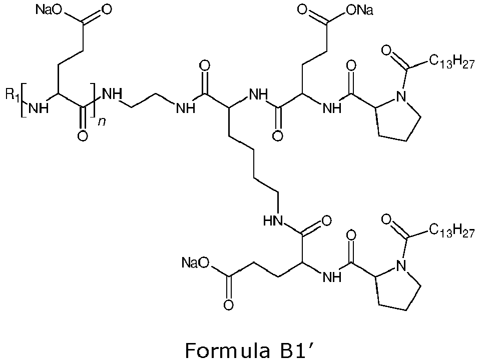 with R1 = H or pyroglutamate, DP is comprised between 15 to 50, in particular 20 to 30.

In an embodiment the co-polyamino acid correspond to the Compound B1 or B22 from WO2019110788.

In an embodiment, the concentration of co-polyamino acid ranges from 1 to 10 mg/ml.

In an embodiment, the concentration of co-polyamino acid ranges from 1.5 to 5 mg/ml.

In one embodiment, the co-polyamino acid/pramlintide molar ratio is greater than or equal to 1.

In one embodiment, the co-polyamino acid/pramlintide molar ratios are comprised from 1.5 to 75.

In one embodiment, the co-polyamino acid/pramlintide molar ratios are comprised from 2 to 50.

In one embodiment, the co-polyamino acid/pramlintide molar ratios are comprised from 3 to 30.

In one embodiment, the co-polyamino acid/pramlintide molar ratios are comprised from 4 to 30.

In one embodiment, the co-polyamino acid/pramlintide molar ratios are comprised from 5 to 30.

In one embodiment, the co-polyamino acid/pramlintide molar ratios are comprised from 8 to 30.

In one embodiment, the co-polyamino acid/pramlintide molar ratios are comprised from 10 to 30.

In one embodiment, the hydrophobic radical Hy/pramlintide molar ratios are comprised from 1.5 to 60.

In one embodiment, the hydrophobic radical Hy/pramlintide molar ratios are comprised from 2 to 60.

In one embodiment, the hydrophobic radical Hy/pramlintide molar ratios are comprised from 3 to 60.

In one embodiment, the hydrophobic radical Hy/pramlintide molar ratios are comprised from 4 to 60.

In one embodiment, the hydrophobic radical Hy/pramlintide molar ratios are comprised from 5 to 60.

In one embodiment, the hydrophobic radical Hy/pramlintide molar ratios are comprised from 8 to 60.

In one embodiment, the hydrophobic radical Hy/pramlintide molar ratios are comprised from 10 and 60.

In an embodiment, the compositions according to the invention moreover include zinc salts at a concentration from 0 to 800 µM per 100 U of insulin.

In an embodiment, the compositions according to the invention moreover include zinc salts at a concentration from 0 to 500 µM per 100 U of insulin.

In an embodiment, the compositions according to the invention moreover include zinc salts at a concentration from 100 to 500 µM per 100 U of insulin.

In an embodiment, the compositions according to the invention moreover include zinc salts at a concentration from 200 to 400 µM per 100 U of insulin.

In an embodiment, the compositions according to the invention moreover include zinc salts at a concentration of 300 µM per 100 U of insulin.

In an embodiment, the compositions according to the invention moreover include buffers.

In an embodiment, the compositions according the invention include a buffer selected from the group consisting of a sodium acetate buffer and Tris.

In an embodiment, the compositions according the invention moreover include preservatives.

In an embodiment, the preservatives are selected from the group consisting of m-cresol and phenol, alone or in a mixture.

In an embodiment, the concentration of preservatives is from 10 to 50 mM.

In an embodiment, the concentration of preservatives is from 10 to 40 mM.

In an embodiment, the compositions according to the invention moreover include a surfactant.

In an embodiment, the surfactant is selected from the group consisting of Poloxamer 188, Tween^{®} 20, also referred to as Polysorbate 20, and Tween^{®} 80, also referred to as Polysorbate 80.

In an embodiment, the Tween^{®} 20 concentration varies from 5 to 50 µg/mL.

In an embodiment, the Tween^{®} 20 concentration varies from 5 to 25 µg/mL.

In an embodiment, the Tween^{®} 20 concentration is 10 µM.

The compositions according to the invention can moreover include additives such as tonicity agents.

In an embodiment, the tonicity agents are selected from the group consisting of glycerol, sodium chloride, mannitol and glycine.

In an embodiment, the compositions according to the invention moreover include an antioxidant.

In an embodiment, the antioxidant is methionine.

The compositions according to the invention can moreover include all the excipients in compliance with the Pharmacopoeias, in particular the EP and/or US Pharmacopoeias, and compatible with the insulins used at the usual concentrations.

According to an embodiment, the composition can be in solid or lyophilized form. This composition can then be used to reconstitute a solution or a formulation.

The methods of administration considered are by the intravenous, subcutaneous, intradermal or intramuscular route.

According to a particular embodiment, the method of administration is the subcutaneous route.

The transdermal, oral, nasal, vaginal, ocular, buccal, pulmonary administration routes are also considered.

The invention also relates to an implantable or transportable pump comprising a composition according to the invention.

The invention also relates to the use of a composition according to the invention which is intended to be placed in an implantable or transportable pump.

In an embodiment, the formulations are in the form of an injectable solution.

The preparation of a composition according to the invention has the advantage that it can be implemented by simple mixing of an aqueous solution of an amylin receptor agonist and of prandial insulin, in an aqueous solution or in lyophilized form.

If necessary, the composition of the mixture is adjusted in terms of excipients such as glycerol, m-cresol, zinc chloride and polysorbate 20 (Tween^{®} 20). This addition can be carried out by addition of concentrated solutions of said excipients.

The compositions of the invention and in the Examples can be obtained via analogous protocols than those disclosed in WO2019020820, WO2021240004, WO2021240006, WO2019110788 or WO2019110797 incorporated by reference.

### Description of the figures:

### Figure 1:

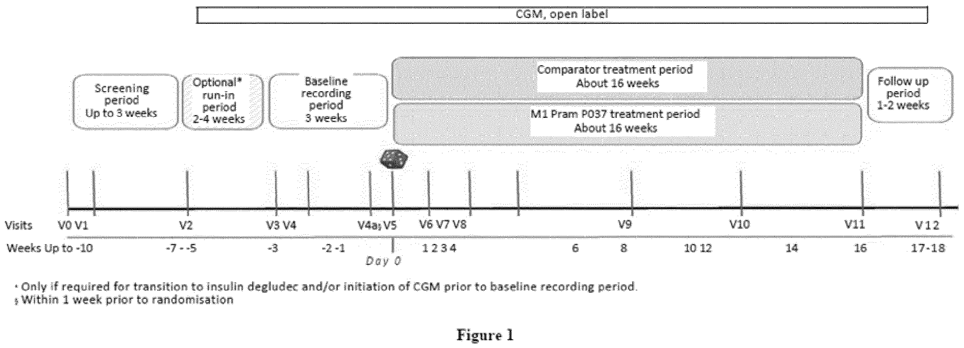

Figure 1 is schematic overview of the Chronological Structure of the Trial

### Examples

### Part A: Product

### Example A1: M1Pram (Composition of human insulin A21G and pramlintide)

The MlPram is an injectable aqueous composition containing 3.45 mg/mL (100 U/mL) of human insulin A21G, 0.6 mg/mL of pramlintide and 18 mM of acetate buffer at pH 4.

This composition can be obtained by analogous process than those disclosed in WO2019020820A2.

### Part B: Clinical Trial

### Example B1: A Phase 2 Trial to Assess the Efficacy and Safety of M1Pram comprising prandial insulin in T1DM Subjects

A phase 2 clinical trial was conducted to confirm the efficacy and safety of MlPram in subjects with type 1 diabetes (NCT04816890).

The MlPram is intended to be injected by the subcutaneous route. In such a formulation, Human insulin A21G shows the pharmacokinetic and pharmacodynamic characteristics of a rapid-acting mealtime insulin, not those of a basal insulin.

### Objectives of the trial:

The primary objective of the trial was to compare the efficacy of prandial use of MlPram to Humalog^{®} (insulin lispro) on body weight change after 16 weeks of optimised titration, in combination with a basal insulin.

In addition, exploratory objectives included the comparison of the efficacy of prandial use of MlPram with Humalog^{®} in subgroups of subjects with a body mass index (BMI) and HbA1c above and below the median at screening.

A post-hoc analysis also included the comparison of the efficacy of prandial use of MlPram with Humalog^{®} in subgroups of subjects with a body mass index (BMI)>=30 kg/m².

### Endpoints of the trial:

The primary endpoint was the body weight change from baseline to week 16 of treatment

In addition, exploratory endpoints included analysis of the subgroups of the primary endpoint according to BMI and HbA1c below and above the median.

A post-hoc analysis was also performed to analyze the subgroups of the primary endpoint according to BMI>=30 kg/m².

### Trial Design:

This was a multicentric, open-label, randomized, two-arm parallel, active-comparator controlled phase 2 clinical trial evaluating the efficacy and safety of MlPram in subjects with type 1 diabetes.

Key inclusion criteria included subjects
- with T1D
- aged between 18 and 64 years
- with a Body Mass Index (BMI) between 25.0 and 35.0 kg/m², both inclusive,
- with HbA1c between 7.0 % and 9.5 %, both inclusive
- with a Diabetes duration of at least 12 months
- Using a multiple dosing insulin therapy (MDI) with a basal insulin and a rapid-acting insulin
- having at least two meals per day.

80 patients were randomized 1:1 to MlPram or Humalog^{®}.

The trial consisted in 4 periods:
- an optional a run-in period of 2 to 4 weeks for patients not already using the Continuous Glucose Monitoring (CGM) system used in the trial or either insulin degludec or insulin glargine as basal insulins
- a 3-week baseline data recording period
- after randomization, a treatment period of about 16 weeks (W1 to 16)
- and a safety follow-up period of 1 to 2 weeks after the last product administration.

The trial design is shown in figure 1.

### Analysis of key Clinical Results:

37 subjects completed the trial in the Humalog^{®} arm and 34 in the M1 Pram arm. The body weight change after 16 weeks of treatment for all completers and for subgroups are presented in the table 1. The subgoups were selected depending on the patients' metrics at screening.

The results indicate that the patients with a higher BMI (above 28 kg/m² or above or equal to 30 kg/m²) benefited more from the M1 Pram treatment in terms of body weight control than the whole population of patients above 25 kg/m².

Similarly, patients that had a worse glycemic control (HbA1c > 7.6%) benefited more from the treatment in terms of body weight control.

**Table 1**

| | M1Pram | | Humalog^{®} | | P |
|---|---|---|---|---|---|
| Group/Subgroup | N | Mean BW change in kg (SD) | N | Mean BW change in kg (SD)) | |
| All completers | 34 | -2.51 (3.01) | 37 | -0.45 (3.12) | p<0.01 |
| BMI<=28 kg/m² | 18 | -1.93 (2.36) | 20 | -0.69 (3.19) | NS |
| BMI>28 kg/m² | 16 | -3.18 (3.57) | 17 | -0.17 (3.09) | p<0.01 |
| BMI>=30 kg/m² | 7 | -5.56 (2.87) | 9 | -0.57 (3.60) | p<0.05 |
| HbA1c<=7.6% | 18 | -1.78 (2.36) | 20 | -0.87 (2.20) | NS |
| HbA1c>7.6% | 16 | -3.34 (3.49) | 17 | 0.04 (3.95) | p<0.01 |

## Claims

1. A composition comprising an insulin and an amylin receptor agonist for treating diabetes, in particular type 1 diabetes, in a patient/person having a BMI of more than 28 kg/m², in particular of more than or equal to 30 kg/m² and/or an HbA1c of more than 7.6 %.

2. A composition according to Claim 1, **characterized in that** the insulin is a prandial insulin.

3. A composition according to Claim 1 or 2 **characterized in that** the insulin is an insulin A21G.

4. A composition according to Claim 2 **characterized in that** the insulin is human insulin A21G.

5. A composition according to Claim 2 **characterized in that** the insulin is insulin lispro A21G.

6. A composition according to Claim 2 **characterized in that** the insulin is insulin aspart A21G.

7. A composition according to claim 1 **characterized in that** the insulin is human insulin, insulin lispro or insulin aspart.

8. A composition according to any one of claim 1 to 7 **characterized in that** it comprises from 40 to 500 U/ml of insulin.

9. A composition according to any one of claim 1 to 8 **characterized in that** the amylin receptor agonist is a short-acting amylin receptor agonist, in particular pramlintide.

10. A composition according to any one of claim 1 to 9 **characterized in that** it comprises from 0.2 to 5 mg/ml of amylin receptor agonist.

11. A composition according to any one of claim 1 to 10 **characterized in that** the composition from 0.3 to 2.5 mg of amylin receptor agonist for 100 U of insulin.

12. A composition according to any one of claims 1 to 11 **characterized in that** it is an aqueous solution.

13. A composition according to claim 12 **characterized in that** comprises an insulin A21G and has a pH ranging from 3.0 to 4.4.

14. A composition according to claim 12 **characterized in that** it comprises a prandial insulin and having a pH ranging from 6.0 to 8.0.

15. A composition according to claim 14 **characterized in that** it further comprises a co-polyaminoacid bearing carboxylate charges and hydrophobic radicals, in particular according to Formula X.
